# EUROPEAN PATENT APPLICATION

(11) **EP 4 556 052 A1**
(43) Date of publication of application: **21.05.2025**
(21) Application number: 23210225.1
(22) Date of filing: 15.11.2023
(51) Int. Cl.: A61M 39/28, A61M 39/08

(54) **DEVICE WITH TWO BODY PARTS ASSEMBLED FOR PINCHING-OFF HOSES**

(71) Applicant: Sartorius Stedim FMT, 13400 Aubagne (FR)
(72) Inventor: Delasalle, Brice, 13600 La Ciotat (FR); Halin, Mickael, 13600 La Ciotat (FR)
(74) Representative: Derambure Conseil

(57) **Abstract**

The clamping device (1) has a body having opposite openings (31, 32) forming a passageway for flexible hose. Two jaws allow a hose clamping, in a closed position maintained at jaw front ends by a tongue (5) engaged with complementary securing means (TS). An upper jaw (11) is downwardly movable to close the device when pushing a top pressing surface (PS). The body has a left body part and a right body part, interconnected at a connector section (CC). The front end (E1) of first jaw comprises the tongue (5) and a hollow upper bulge (B1) offset upwardly. This front end consists of two halves, one of which is arranged at front end of a first connector section belonging to a body part. The first connector section has an insert (41) extending inside said bulge and engaged in a transverse passage of the second half forming the second connector section.

## Description

### FIELD OF THE INVENTION

The instant disclosure relates to fluid management systems, and more particularly to devices for pinching-off flexibles hoses carrying liquid for the purpose of cutting-off and/or regulating the throughflow, this device possessing two interacting stirrups or jaws, between which the hose is nipped.

### TECHNICAL BACKGROUND

Hoses carrying biopharmaceutical fluid are commonly used for interconnecting flexibles pouches or similar parts of a circuit. The term "biopharmaceutical fluid", in the following, is understood to mean a product of biotechnology (culture media, cell cultures, buffer solutions, artificial nutrition liquids, blood products and blood product derivatives) or a pharmaceutical product or more generally a product intended for use in the medical field. The hoses carrying such kind of fluid may be involved for connection to an inlet or outlet of a bioreactor or the like, for instance for single-use applications, in combination with one or more clamps.

Hose clamps, provided with a lower jaw and an upper jaw connected to a common bendable connection, have two opposite axial openings (along a rear-front direction of the clamp) and inwardly protruding pinching parts distributed in the lower and upper jaws. Document US 10,335,586 B2 shows a clamp that can be locked in a closed position with a pinching of the hose, thus stopping fluid communication through the hose segment received in the clamp. A drawback with such construction is that, for the positioning of the clamp, a slide-on installation from the end of the hose or tube is required. The need for a clamp must be anticipated.

More generally with current constructions, the clamp must be installed at assembling phase of the kit, not later (during operation). It also requires cutting pipes to take the clamp out for recycling.
In document WO 0044434 A1, a squeeze clamp has also been proposed with lateral slots to permit a flexible tube to be positioned through spaced axial openings of the squeeze clamp. This allows the clamp to be subsequently added to a manufactured tubing set or circuit when desired. However, the structure of the clamp is weakened, and pinching efficiency may be altered.

There remains still room for improvement regarding clamping devices that may be robust, efficient, while providing flexibility of use.

### OBJECTS AND SUMMARY

For improving situation, embodiments of the invention provide a clamping device for regulating the flow of liquid passing through a flexible hose, comprising a clamp body having a longitudinal axis defining a front-rear direction, the clamp body comprising:
- a rear opening and a front opening, which are opposite along the longitudinal axis and are forming a hose passageway for receiving the flexible hose;
- a pair of clamping jaws each provided with a clamping part protruding in the hose passageway in a closed position of the clamping device;

wherein interacting securing elements are distributed at front clamp ends of the clamping jaws and are mutually engaged to anchor the clamping jaws in the closed position, the clamping jaws comprising a first clamping jaw and a second clamping jaw extending both from a bendable connection to a corresponding front clamp end, the first clamping jaw having a pressing surface for actuation of the device and being downwardly movable in response to a pushing action at the pressing surface that is facing upwardly and formed in an upper outer face of the clamping device;
wherein the clamp body is an assembly consisting of a first body part and a second body part connectable/interconnected by fastening means that are offset relative to the hose passageway,
and wherein the (front) clamp end of the first clamping jaw, forming a first clamp end, comprises a tongue and a hollow upper bulge offset upwardly relative to the tongue, the first clamp end having two complementary parts or halves with one half arranged at a front end of a first connector section (belonging to the first body part) and the other half arranged at a front end of a second connector section (provided in the second body part) that delimits a transverse passage, the first connector section including an insert configured to project inside a hollow region of the second connector section via the transverse passage so that the insert extends inside said upper bulge.

It is understood the first and second body parts form or constitute a left body part and a right body part, allowing delimiting left sides and right sides, respectively, of the rear and front openings once the first body part and the second body part are assembled together, the clamp body including a longitudinal junction for junction of the first and second body parts in an assembled state that can be obtained after placing a hose segment of the flexible hose in an interspace between the left sides (rear left side and front left side) and right sides (rear right side and front right side).

The tongue is protruding at the opposite from the rear opening and is one of the interacting securing elements. Since the first connector section belong to the first body part and the second connector section belongs to the second body part, the tongue and the bulge are also distributed in the first and second body parts.

With such arrangement, the clamping device can be assembled with suitable position along a tube/hose, when desired, while ensuring an efficient clamping. Actuation is simple, the first clamping jaw defining an upper actuatable portion of the clamp body. Connection between the two body parts is robust, using an enlarged front end of the upper/first clamping jaw where a connecting insert extends (insert that protrudes laterally in one of the body halves/parts). Interconnection between clamp halves may thus use several interconnecting members, for instance at least in the two front ends of the jaws, typically with also a connection performed at location of a lower clamping part (provided in the second clamping jaw), with the larger connection realized in the first clamping jaw near the tongue, for example just below the pression surface and adjacent to a front portion of the bulge.

According to an embodiment, the bendable connection may be arranged at a clamping device axial end provided with the rear opening. Typically, the clamping device is forming a closed loop in the closed/closing position (as seen in a side view), and an open loop in expanded position (with the loop opened at the opposite from the rear opening).

The two clamp ends comprise a first clamp end and a second clamp end, which are adapted to constitute the two ends of the open loop.

The insert and other interconnecting elements of the fastening means may be distributed above and below the passageway, in expanded state (open state/position of the clamping device). In embodiments, the fastening means may be radially offset relative to the hose passageway, with the fastening means preferably concealed in assembled configuration of the clamping device. The junction for assembled state may be a discontinuous junction, to obtain the rear and front openings, possibly without creating any recess opening radially in the hose passageway at other locations.

The tongue is rigid and protrude forward, away from the fastening region of the connector section (connector). The connector section, obtained by interconnecting the first connector section and the second connector section, involves a rigid connection, without any relative movement allowed between the body parts. In some options, the tongue cooperates with a toothed section. The wording "toothed section" here encompasses any configuration with ribs (ribbed section) suitably forming notches for engagement of the tongue with a retaining/locking effect.

In some embodiments, the clamping device is provided with one or more of the following features:
- the first clamp end is included in a connector consisting of the first and second connector sections in assembled state.
- the connector has a generally annular or rectangular periphery with lower and upper elongated sides.
- the insert forms an insertion member that is hollow, with at least one open end, the insertion member being preferably of annular shape.
- the transverse passage is delimited between the lower and upper elongated sides.
- the transverse passage opens at two opposite sides (which are right and left sides) of the body half provided with the second connector section.
- the lower elongated side of the connector extends from the clamping part of the first clamping jaw to the tongue (for instance a base of the tongue may be arranged at or adjacent to such lower elongated side).
- the insert comprises two lugs or is of annular shape (with the lugs or annular wall of the insert extending perpendicular to the hose passageway), so that the insert delimits inside the insert an inner hollow space that opens on at least one side of the clamping device,
and wherein the connector, which provides in assembled state the upper pressing surface for actuating a closed position of the clamping device, is an annular connector, preferably longitudinally elongated, with an annular shape as viewed from a side of the clamping device.

In some options, a junction between the two body parts defines a junction plane, with the connector section selectively distributed on either side of the junction plane, only the insert extending beyond this junction plane. In some variants, the junction may correspond to a plane only for the lower clamping jaw, for instance with two fastening regions provided in the lower clamping jaw, while the connector including the insert may form a connector junction that is orientated slightly differently.

According to a particular embodiment, the first clamping jaw (forming an upper jaw under usual handling conditions) comprises a camber region adjacent to a rear end of the connector (thus adjacent to fastening region using the insert). The camber region may be arranged between the rear opening and the first connector section. This camber region may extend between the rear opening and a connector rear end included in the connector formed by the first connector section and the second connector section.

The camber region may provide a deviation allowing the connector to extend upwardly from a direction of the longitudinal axis. Such deviation provides a pressing surface progressively extending upwardly with increased space from the camber region, the pressing surface being preferably a planar or flat surface.

With such deviation, the bulge (which may be a boss or lump) may be formed, at least partly, at location of the pressing surface. The camber may be adjacent to an annular section of the first clamping jaw. The camber thus may be provided between the left and right sides delimiting the rear opening and between a junction of an upper branch of the first clamping jaw and a lower branch forming the first clamping part, with both branches extending rearwardly from the front clamp end.

The insert may be tilted due to the deviation, so that the insert extends above the rear opening in expanded state.

The insert, in the first section/male section of the connector, can be as wide/high as a height of a cavity or hollow of the female section/first section of the connector. A hollow structure of the insert may be chosen, possibly with the insert protruding from a ring part of the first connector. The ring part may have two parallel sides, forming an upper side with the pressing surface and a lower side that joins the first clamping part.

It is understood that the connector in the first clamping jaw may form a ring, thus providing a hollow region where the insert extends along a transverse direction (typically left-right direction).

Above the hollow region provided in the first clamping jaw, the upper outer face provides the pressing surface suitable to be pushed downwardly to obtain the closed position of the clamping device. In the closed position, a hose segment of the flexible hose passing through the two openings (rear and front openings) can be nipped between the first and second clamping parts.

Whatever the way the body parts are connected, the insert may have an insertion size lower than an insert length as measured along a direction of the first clamping jaw going away from the rear opening. The insert length may be higher than 9 mm, for instance higher than 14 mm. When the insert is/acts as a clip, this may ensure that abutment length is sufficient to prevent any accidental disconnection.

The clamp body can be made of two pieces, for instance two plastic pieces, and may comprise three pairs of interlocking elements for securing the two pieces that form the first and second body parts. One of the pairs includes the insert and the transverse passage (with this transverse passage formed in the second connector section opening at a joining face of the second body part). The bendable connection and delimitations (left and right sides) of the two openings are distributed in the two pieces, so that the two pieces can be fastened using the interlocking elements to obtain the clamping device once the flexible hose is already extending between the two pieces. In assembled state, the hose is positioned in the hose passageway.

The junction may be provided at a median plane including a longitudinal axis of the clamping device. Possibly, one or more slots may be provided at such junction to allow insertion/use of a tool to ease reopening (to separate the two pieces/ body parts). A slot may be elongated and provided along such junction at the first clamping jaw and/or at the second clamping jaw. Of course, the junction may also be provided at an offset plane, i.e. a plane that is offset on the right of left of the device: for instance the first connector section may be of greater width than the second connector section. Also, the first body part may have a width, as measured in the clamping subparts along a transverse left-right direction, greater than complementary width of the second body part, to obtain such offset junction (with an offset longitudinal planar junction).

The longitudinal junction, possibly provided at an offset plane (offset relative to a symmetry plane), can be an asymmetric junction so that a body part amongst the first body part and the second body part has:
- a U-shaped circumference part at the rear opening and at the front opening, preferably with at least two non-circular segments that define two opposite branches in the U-shaped circumference;
- a width that is greater than 55% of a full width of the clamping device, as measured along a left-right direction.

Use of rear access to side lugs or reliefs provided in the insert, due to the hollow region in the first connector section, allow a disconnection, typically with help of a suitable tool. Also, recesses that create such access may allow an increased compressibility of plastic material present in lugs or similar reliefs of the insert engaged in the second connector section, which make locking the connection easier, with a possibility of disconnection, for instance when abutment surface are slanted to allow to facilitate disengagement by exerting an action in direction opposite to insertion direction.

In some options, the two clamp ends (for the two clamping jaws) are offset the hose passageway, the second clamp end including a securing surface with at least one retaining rib or tooth facing inwardly, while the first clamp end, provided with the tongue to be engaged with said securing surface, is actuatable to allow the hose pinching. In expanded state of the clamping device, the tongue is transverse (i.e. orientated transverse and offset) to the securing surface and can protrude outwardly, without extending above an upper portion of the insert (in contrast, the tongue can be at a lower end of a front face of the first clamp end.

According to embodiments, at least the first clamp end in the first clamping jaw is locally enlarged or as thick as the first clamping part. Optionally, all or part of the clamp ends may be enlargements of the clamping jaws.

In particular embodiments, the clamp end of the second clamping jaw constitutes a second clamp end, the first clamp end being:
- configured to interact with a rear surface of the second clamp end by the tongue to anchor the clamping jaws in the closed position, the tongue being preferably secured to a toothed section forming the rear surface of the second clamped end.

Optionally, the clamping part of the first clamping jaw is:
- a first clamping part protruding downwardly; and
- provided between the rear opening and the first clamp end that is arranged in the first clamping jaw at the opposite from the bendable connection.

The first clamp end may comprise a front face with the tongue protruding forward from a lower portion of the front face.

Besides, the second clamping jaw, which is provided with the front opening, may define a lower portion of the clamp body. The front opening can extend between a second clamping part (facing the first clamping part) and a second clamp end that is arranged in the second clamping jaw. Typically, the second clamping part forming the clamping part of the second clamping jaw is protruding upwardly toward the first clamping part. The first clamping part may be included in the connector, so that the first clamping part is an assembled subpart of the first connector section and the second connector section.

Geometry of the insert may vary, the insertion area being elongated and preferably corresponding to a single cavity or passage for the insert, without any partitioning wall inside the second connector section. The insert protrudes and extends (possibly with a protruding distance superior or equal to 4 or 5 mm) along an insert direction. Protrusion extension is lower than a longitudinal extent of the insert. The insert may have an annular cross section as viewed in a longitudinal plane parallel to the junction.

In a first embodiment, with such annular cross section, the insert can optionally have an annular bead (peripheral bead) configured to be retained in the second connector section, in the assembled state, by one or more abutment surfaces protruding inwardly to locally narrow an inner circumference of the second connector section that is annular.

In a second embodiment, the insert of annular cross section is provided with several lugs configured to be retained, in the assembled state, by one or more abutment surfaces protruding inwardly to locally narrow an inner circumference of the second connector section that is annular.

In a third embodiment, the insert includes two spaced clips that preferably have same protruding extension. The spacing allow the clips to be flexed when being engaged against complementary inner reliefs of the second connector section. The inner reliefs form abutment surfaces protruding inwardly to locally narrow an inner circumference of the second connector section. When the insert is fully inserted, each clip extends beyond a corresponding abutment surface and is retained in the transverse passage.

In embodiment of the device, one or more of the following features may be provided for the insert that provides fixation beneath the pressing surface:
- the insert is protruding beyond an inner shoulder provided in the second connector section.
- the insert has two parallel sidewall members, each provided with a lug adapted to be engaged in abutment against a complementary inner shoulder provided in the second connector section.
- one or more slots may be provided at the inner shoulder, allowing access to lugs of the insert from a side of the first connector section which is at the opposite from a protruding direction of the insert.
- the insert has a width and an insertion size lower than a longitudinal extension of the insert, which is a size of the insert measured along the pressing surface, preferably measured parallel to the pressing surface.
- the insert is extending parallel to the pressing surface, for instance so that the insert has an upper insert face extending parallel to the pressing surface.
- a width (forming height) of the insert in a longitudinal plane is superior or equal to an interspace height, measured perpendicular to the longitudinal axis, corresponding to/being the minimal spacing delimited between the clamping parts in expanded state of the clamping device (the clamping parts being distributed in the first clamping jaw and the second clamping jaw).
- width (forming height) of the insert in a longitudinal plane may be constant and the insert has an oblong cross-section or two spaced elongated straight portions spaced by a constant spacing.

In some options, the clamping device is provided with the securing elements (including the tongue) that are releasable. The two clamp ends are spaced attachment ends in the expanded state, which is an open state of the clamping device with these ends each including a transverse insertion member, the insertion members having same orientation. The insert in the first connector section, with a hollow construction, may at least three times more elongated, along longitudinal direction of the device, than the insertion member present in the second clamp end.

According to another aspect, embodiments provide a use of the clamping device as above presented, wherein a hose/tube(possibly a catheter) forming a lumen for passage of a biopharmaceutical fluid is installed to seat against two spaced guiding regions provided in the first body part, so that the insert protrudes transversally relative to a hose segment of the hose extending between the two spaced guiding regions,
and wherein the second connector section, included in the second body part, is fastened to the first connector section to obtain the assembled state, with the hose extending through the front opening and the rear opening of the clamping device, one of the two spaced guiding regions providing a first perimeter portion, forming a perimeter fraction of the front opening, the other one of the two spaced guiding regions providing a second perimeter portion forming a perimeter fraction of the rear opening, each perimeter fraction representing preferably more than 55% of the opening circumference (full perimeter), respectively for the front opening and the rear opening.

With the fast assembling of the clamping device, such operation is easy and the body parts may be rigidly secured to provide a 360° circumference for each of the axial openings (front/rear opening), thus preventing any radial access or slot to be present adjacent such an axil opening. A clamping action may be performed at later stage by pressing the first clamping jaw, with the clamping parts extending between the two openings, thus at intermediary axial position between the two spaced guiding regions. The bulge, shifting the pressing surface upwardly, is also oh help to prevent any interference/contact between user's finger(s) and the tongue that is significantly offset relative to the outer upper face (uppermost face) of the clamping device.

The pressing surface may extend between a camber region and a front face angled relative to the pressing surface, with the front face extending upwardly sufficiently to cause the deviation angle at the camber region to be higher than 15° or 25°.

### BRIEF DESCRIPTION OF THE DRAWINGS

The figures of the drawings are now briefly described.
Fig. 1 is a perspective view of a first embodiment, in expanded state, of a device suitable for pinching-off a flexible hose carrying fluid.
Figs 2A and 2B illustrate two exemplary body halves suitable to obtain a clamping device with a junction plane that is a median longitudinal plane intersecting the pressing surface.
Figs 3A, 3B and 3C are views with a cut, showing respective parts of the clamping device of Fig. 1 in expanded state.
Figs 4A-4B are respective section-views along a longitudinal plane, showing a second body part the device, which includes the second connector section, in assembled state of the device so that some protruding members of the first body part are also apparent.
Fig. 5 is a perspective detail view, in expanded state, of a device suitable for pinching-off a flexible hose carrying fluid, using two pieces/halves with the insert of a first connector section split into two protruding parts.
Fig. 6 illustrates a part of a circuit for biopharmaceutical fluid, where a clamping device according to invention can be assembled when desired and then closed by pressing the upper bulged part.
Fig. 7 illustrates, by a perspective view, a clamping device have a junction plane that is offset to obtain a first body half of greater width than the second body half, which is of interest for pre-positioning a hose before assembling the clamping device.
Fig. 8 illustrates, by a top view, the clamping device of Fig. 7, showing the tongue and a top of the upper bulge that increases thickness (height) of the attachment end including the tongue.
Fig. 9A illustrates, just before the assembling operation, two complementary body parts allowing forming a compact clamping device.
Fig. 9B is a rear view showing a clamping device similar to the device of Fig. 9 A but provided with guiding branches for adjustment to different hose sizes.
Fig. 10 is a side view of the clamping device in closed position available amongst several closing positions, with the hosed pinched between the rear and front openings.
Fig. 11A shows two halves of the clamping device that can be assembled, along a longitudinal junction plane, using interlocking means with clips for a definitive fastening, while Fig. 11B show a variant of reopenable device including protruding extensions of the clips.
Fig. 12 is a perspective view of a device similar to the example of Figs 9A-9B, but with a hinge connection provided for making the body parts inseparable.

### MORE DETAILED DESCRIPTION

A detailed description of several embodiments of the invention is provided below, accompanied with examples and with reference to the drawings.

In the various figures, the same references are used to designate identical or similar elements. In the various figures, the same references are used to designate identical or similar elements.

Thickness size is to be understood in a usual way for the skilled person (thickness can be measured in a longitudinal plane/longitudinal cut view of the clamping device, intersecting a pressing surface), while width reflects transverse size (along a right-left direction) measured perpendicular to such longitudinal plane.

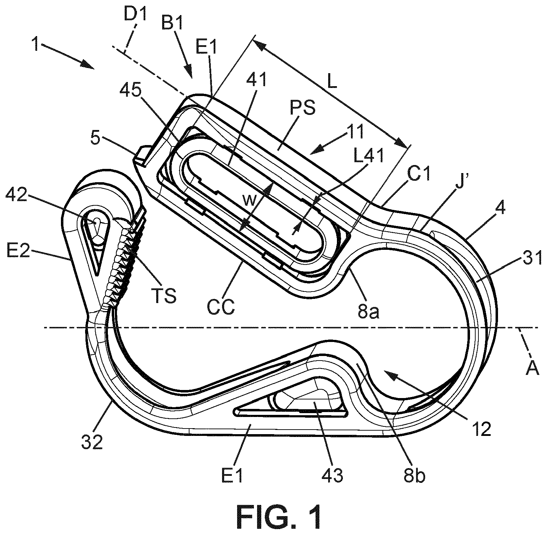

Referring to Fig. 1, 2A-2B and 9A-9b, it is shown exemplary construction of a hose clamping device 1, possibly made of two pieces of plastic material. The device 1, once assembled by interconnecting the two pieces, may comprise two clamping jaws 11, 12. These jaws 11, 12 both extend to respective front clamp ends, at the opposite from a bendable connection 4, as well apparent in Fig. 1. The jaws 11, 12 are provided in a same clamp body having a longitudinal axis A defining a front-rear direction. The upper jaw is a first clamping jaw 11 that is actuatable to reach a closed position of the device 1. Actuation may be obtained by a pushing action exerted on a pressing surface PS that is formed on an upper outer face F2 of the clamping device 1.

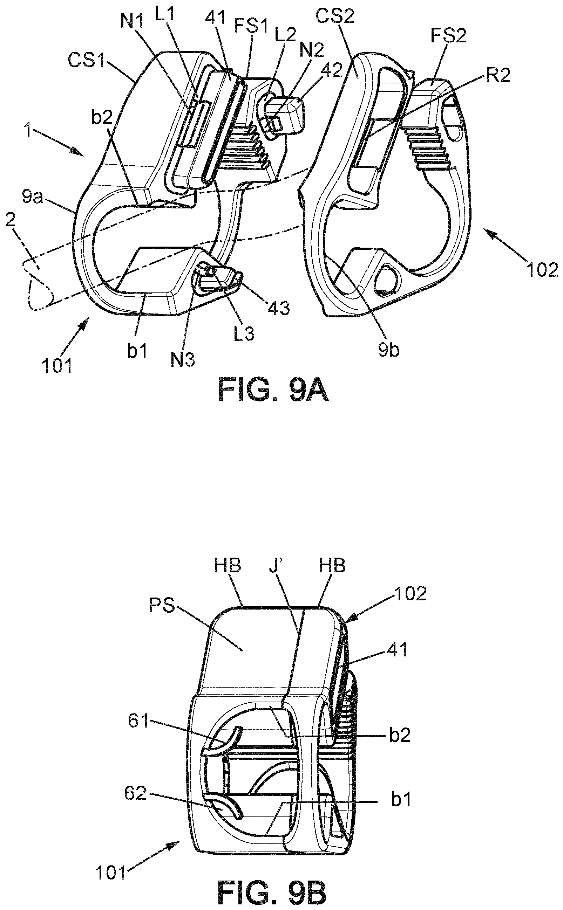

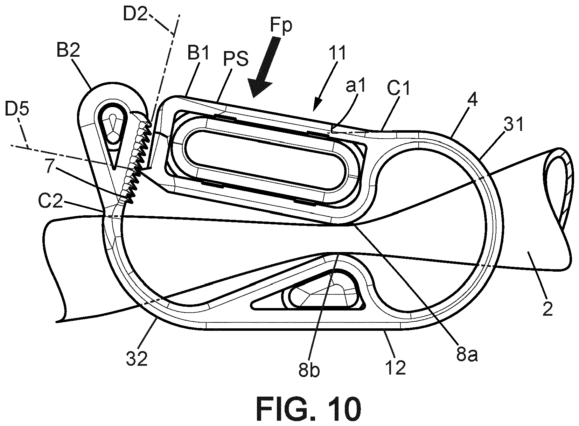

Referring to Figs 9A-9B and 10, when a hose 2 is placed between the two clamping jaws 11, 12, selective displacement of the first clamping jaw 11 along pushing direction Fp causes the pinching of the hose 2 between two clamping parts 8a, 8b distributed in the upper and lower clamping jaws 11, 12. In order to have the hose segment of the hose 2 maintained/stabilized in a hose passageway delimited between the jaws 11, 12, the clamp body has a rear opening 31 and a front opening 32, which are arranged opposite along the longitudinal axis A, thus forming the hose passageway. Circumferences of these openings 31, 32 may be of circular section or of generally annular section. A geometry with straight edges or similar edges may be provided, in order to locally reduce the opening circumference (at opening 31 and/or at opening 32) and providing ability to retain the hose 2 already by one of the two pieces (body parts 101, 102 as described in more detail below). Of course, these circumferences may have any suitable section matching or partly matching with hose section, for instance with a half circumference substantially circular. The clamping parts 8a, 8b may protrude in the hose passageway, at an intermediary space between the openings 31 and 32, in a closed position of the clamping device 1. For instance, the second clamping part 8b provided in the second clamping jaw protrudes upwardly in the hose passageway, already in expanded state of the device such as illustrated in Fig. 1. The first clamping part 8a is protruding downwardly and may be offset upwardly in the expanded state, with a first clamp end E1 included in the first clamping jaws 11 facing forward and upward. With such configuration, the first clamp end E1 is a front end of the jaw 11 which is spaced relative to a second clamp end E2 formed at the front of the device 1 and included in the second clamping jaw 12.

The closed position of the clamping device 1 may be locked thanks to securing elements. Referring to Fig. 1, the pair of clamping jaws 11, 12 are provided with interacting securing elements 5, TS distributed at the front clamp ends E1, E2 of the clamping jaws 11, 12. The securing elements 5, TS can be mutually engaged to anchor the first and second clamping jaws 11, 12 in the closed position. In the first clamp end E1, the securing element may be a tooth or tongue 5 that protrudes outward, at the opposite from the rear opening 31. In the second clamp end E2, the securing element may include one or more ribs, for instance forming a toothed section TS that is facing inward, i.e. toward the rear opening 31. Of course, any suitable rear surface may be used to form a securing element in the second clamp end E2, interacting with the lowered securing element (tongue 5 or the like) protruding forwardly at the first clamp end E1.

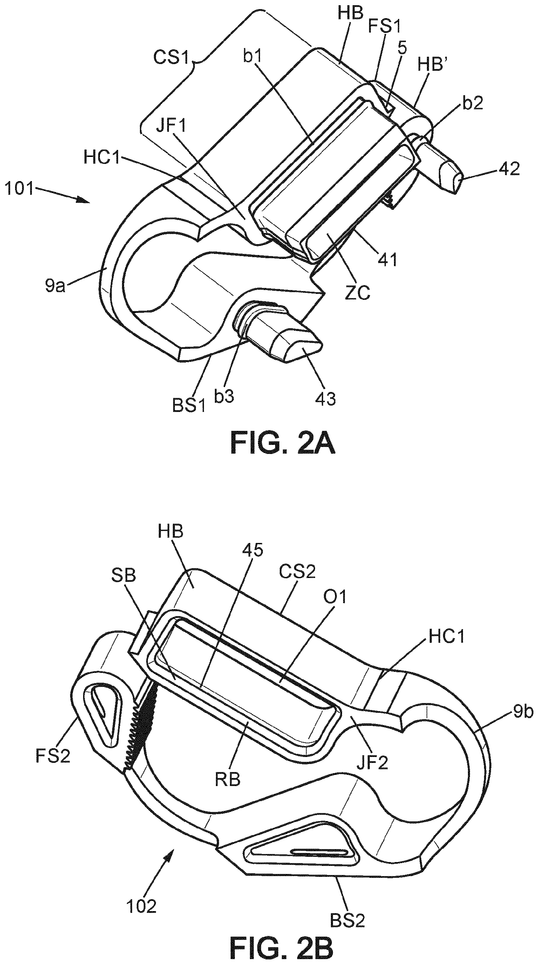

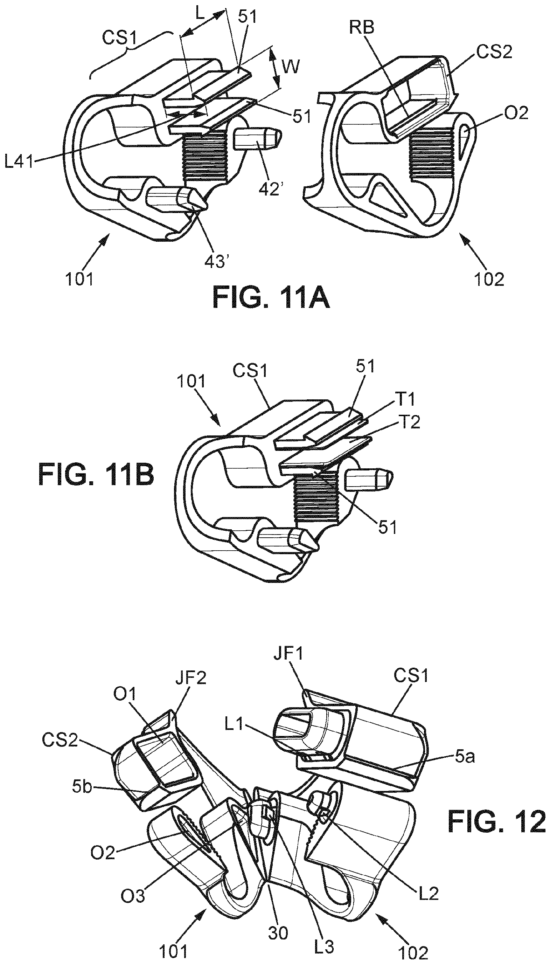

As illustrated in Figs 2A-2B, 9A, 11A and 12, the clamp body has an assembled state obtained by interconnecting two body parts 101, 102 that may be initially separated (made separately) or partially linked, for instance using a hinge connection 30 (as apparent in Fig. 12) or similar link. In any case, the construction of the two body parts 101, 102 allow the hose 2 to be introduced along an inner face of a body part that defines sides of the openings 31, 32, for instance along the first body part 101, before being housed in the hose passageway in the assembled state where a junction J, J' is formed to have the body parts 101, 102 mutually adjacent and typically extending parallel to a longitudinal plane that intersects the pressing surface PS.

As apparent from Figs 2A-2B, 5 and 7-8, the left and right body parts 101, 102, each have:
- a connector section CS1, CS2, provided with a tongue segment 5a, 5b protruding along direction D5 (protruding direction of the tongue 5) and a downwardly protruding member of the first clamping part 8a;
- a first branch 9a, 9b that is arched and laterally offset (to be distant) relative to junction plane P of the device 1, the first branch 9a, 9b (continuous branch) being connected at a rear end of the corresponding connector section CS1 or CS2;
- a base segment BS1, BS2 adapted to form half or fraction of the second clamping part 8b;
- a front segment FS1, FS2, each including a retaining surface portion to allow forming a toothed section TS or similar securing surface with teeth or ribs 7 in assembled state;
- a second branch that is arched to connect the (lower) base segment BS1, BS2 to a corresponding front segment FS1, FS2.

The junction plane P may be a longitudinal median plane so that the two body parts 101, 102 have same width (without taking account the protruding/insertion members that form part of the interlocking members). Alternatively, the junction plane P may be offset to obtain a body part of higher width, which may facilitate pre-positioning of a hose 2. More generally, the longitudinal junction can be an asymmetric junction to have a wider body part. In non-limiting embodiment of Fig. 9A, a hose segment is illustrated, starting to be positioned along a channel provided by the first body part 101 whose width is greater than width of the second body part 102. The first body part 101 is thus typically larger than half diameter of the hose 2.

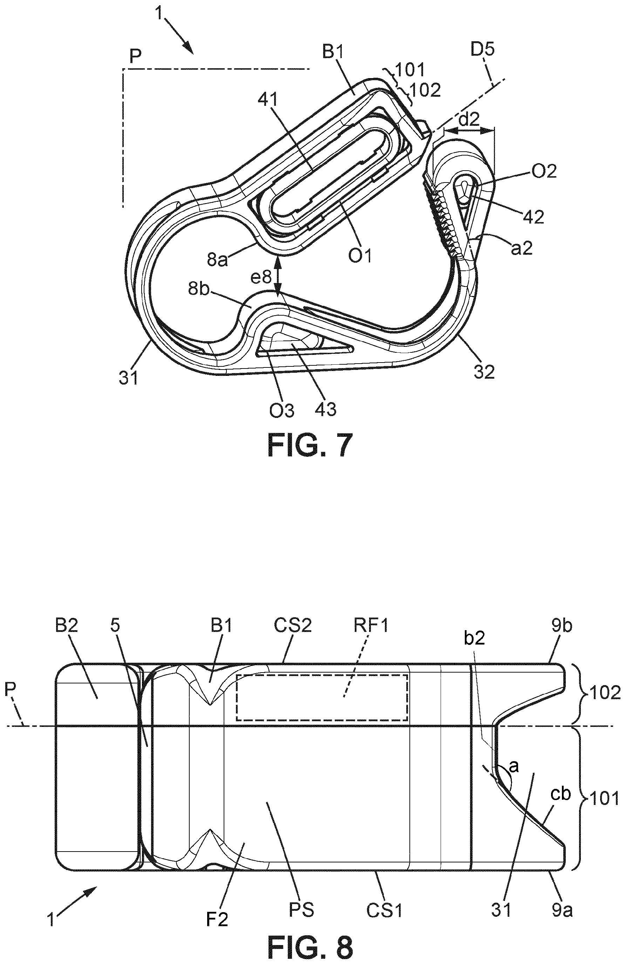

When having an offset junction plane P, the first body part 101 can form respective perimeter fractions for the openings 31, 32 of the clamping device 1, possibly representing more than/at least 55% or 60% of the opening circumference, respectively for the rear opening 31 and the front opening 32. Referring to Fig. 8 and 9A, the rear opening 31 may have at least two edges b1, b2, for instance two opposite edges, possibly straight edges that can act as gripping edges for the hose 2 of circular section. An angle a may be provided at an end of such edges b1, b2, as considered in a top view of the device 1, to have each straight edge b1, b2 (or any suitable edge elongated along left-right direction) angled relative to an adjacent circumference segment. As compared to a circular circumference with constant radius, such arrangement allows locally reducing height of the corresponding opening, for instance at least the rear opening 31. The branch 9a may be U-shaped.

The two edges b1, b2 can be opposite chord segments in a generally annular circumference delimiting the rear opening 31. It is understood the two edges b1, b2 may be adjacent to a same circular segment cb. More generally they are arranged to:
- locally reduce section of the passage at the rear opening 31;
- providing a lowermost hose access size in a body part, for instance at the rear end of such body part 101 that becomes suitable for preventing lateral movement of the hose once inserted in the first body part 101 via such rear end.

These edges b1, b2, here parallel, are included in the first body part 101 so that they allow a gripping or pinching effect to provisionally retain the hose 2 inside the first body part 101 before fastening the other second body part 102. While the insert 41 is illustrated here as being included in the first body part 101 provided with the edges b1, b2, other options with an insert arranged in the complementary part 102 may be provided. The front opening 32 may have a similar or identical circumference arrangement, with two opposite edges that are elongated along a left-right direction in the clamping device 1.

In some options, the rear opening 31 may extend higher than the front opening 32, whereby a hose 2 passing through the respective openings 31 and 32 may be slightly tilted forward (see Fig. 9A for instance). Such tilted configuration may be obtained already when blocking the hose 2 by the first body part 101 or any suitable wider body part, before the assembling of the device 1.

A pre-positioning is obtained after seating the hose segment against the branch 9a and below the first front section FS1. Such pre-positioning may be of help to provisionally maintain the hose 2 without any contact between the hose 2 and the second body part 102 that will be then handled to be fastened to the first body part 101 around the hose segment.

Optionally the tongue 5 may have a junction J5 that is included in the junction plane P, the left and right tongue segments 5a, 5b can have same profile. More generally any of the segments in the body parts 101, 102 may have same profile for the left segment and the right segment. Preferably, the clamping device has no protruding relief/local protrusion along the junction J, J'.

In assembled state with the body parts 101, 102 adjacent and joined along the junction plane P, the first and second connector sections CS1, CS2 form a connector providing the upper outer face F2 of the device 1. As shown in Fig. 1, a camber region C1 may be provided, adjacent this face F2, to have the upper outer face F2 being sloped with the device 1 more and more expanded (upwardly) at the first clamping jaw 11 with increased spaced from the sides or branches 9a, 9b delimiting the rear opening 31. Respective cambers HC1 may be provided in each of the body parts 101, 102 to have a single camber region C1 that is ergonomic, for instance for grasping the device and naturally placing a thumb end near the bulge B1.

The pressing surface PS may be offset upwardly with formation of an upper bulge B1. The lower face of the base (BS1, BS2) and the upper outer face may optionally be arranged with an angle in expanded state: these faces may taper toward the rear opening 31.

Each of the clamping jaws 11, 12 may have an outer face provided with camber C1, C2, such cambers C1, C2 being adjacent to bulged parts of these jaws, so that they are adjacent to a transition section where thickness in the jaw 11, 12 greatly varies (increases). Also, each camber C1, C2 may be associated to a respective deviation angle a1, a2. Each deviation angle a1, a2 may be superior or equal to 15 or 25°.

The first camber C1 provided in outer face of the first clamping jaw 11 may be arranged between the rear opening 31 and a first outer boss or upper bulge B1 of the clamping device. Such bulge B1 has a front portion and is adjacent to the tongue 5, which is formed at a lower portion of a front face F1 of the first clamp end E1. The first connector section CS1 has a half bulge or partial bulge HB, while the second connector section CS2 has a half bulge or partial bulge HB' (as apparent in Fig. 2A, 9A and other figures).

The second camber C2 provided in outer face of the second clamping jaw 12 may be located between the second passage 32 and an optional second outer boss/bulge B2 provided in the second clamp end E2, as apparent in Figs 8 and 10 for instance. With such cambers C1, C2, an expanding effect is obtained, allowing a greater sizing of the respective clamp ends E1, E2 while keeping the gap between these ends E1, E2 before actuating closure of the device 1.

As illustrated in Figs 1 and 8, there is an increase in thickness to define the clamp ends E1, E2. A fixing region RF1, where an anchoring is obtained to fasten the first and second connector sections together, can be located in the second connector section CS2. A top of the fixing region RF1 may be included in or adjacent to a device upper outer wall part that delimits a section (here a right section) of the pressing surface PS. It can be seen the top of the fixing region RF1, typically offset relative to the junction plane P, may be longer than wide.

More generally, at least the first clamp end E1, possibly each clamp end E1, E2, may optionally be provided with a loop profile, thus each having a hollow. More generally, the clamp end E1 may be thick/enlarged with the upper bulge B1 allowing an increase of the distance between the tongue 5 and the pressing surface PS. The bulge B1 is offset upwardly relative to the tongue 5, as well apparent in Figs 1 and 8 for instance.

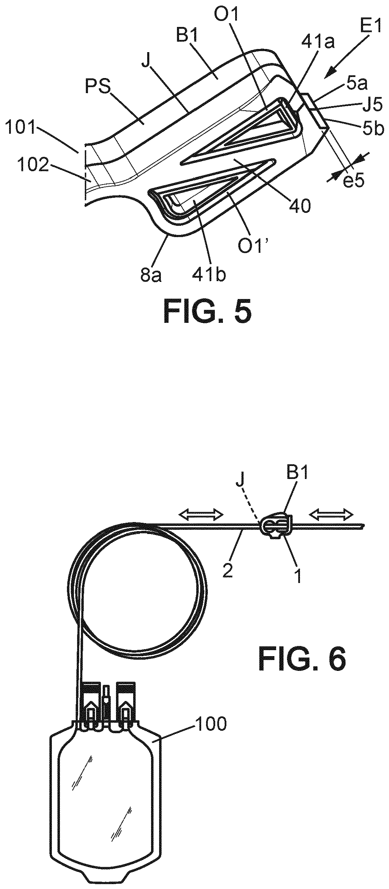

The first clamp end E1, which comprises the tongue 5 and the hollow upper bulge B, can be composed of two halves included in the body parts 101, 102. One first clamp end half is arranged at a front end of the first connector section CS1, while the other first clamp half is arranged at a front end of the second connector section CS2. A transverse passage O1, which opens to the junction side, is delimited by the second connector section CS2. The first connector section CS1 includes an insert 41, 41a configured to project inside a hollow region of the second connector section CS2 via the transverse passage O1. Possibly, one or more additional passages O1' may be formed in the second connector section CS2, typically away from the bulge B1. Figure 5 shows an embodiment where two transverse passages O1, O1' are delimited in the body part 102, with the transverse passage O1 arranged adjacent to the pressing surface PS and the other passage O1' (parallel to the passage O1) located adjacent to the clamping part 8a.

As illustrated in Figs 2A-2C, 3A-3B, 7-12, the two body parts 101, 102 may be two pieces having a form-locking fastening distributed in at least two areas, possibly three areas. The interlocking elements 41, 42, 43, O1, O2, O3 may be arranged in the thickness of the clamping device 1, without interfering with the hose passageway, thus without extending in longitudinal alignment with the openings 31, 32. In the non-limiting depicted embodiments, the interlocking elements 41, 42, 43, O1, O2, O3 comprises at least two pairs of interlocking members that are distributed in:
- the connector CC forming the first clamp end E1; and
- the second clamp end E2.

In preferred options, a pair of interlocking members are also distributed in the base segments BS1, BS2 forming the lower clamping part 8b.

Of course, downward displacement of the first clamping jaw 11 in response to a pushing action on pressing surface PS (action exerted along direction Fp of pressing as shown in Fig. 10 in particular) is simple and the body parts 101, 102 remain interconnected, possibly thanks to at least two or three clips provided in the first connector section. At least two clips 51 or clipping lugs L1 may be provided in the insert 41, 41a, 41b, according to some embodiments.

The clamping part 8a is included in the connector CC, protruding at the opposite from the upper bulge B1. The complementary clamping part 8b may be split in two and be distributed in the two base segments BS1, BS2. Typically, the two upper portions of the base segments BS1, BS2 are separated from a lowermost side of the clamping device 1 by a fastening area where an insertion member 43 is located. The insertion member 43 is included in a body part (for instance the first body part 101) and can protrude transversely inside the lower clamping jaw 12 of the clamping device 1. A lower auxiliary connector is formed, at the opposite from the connector that may be arranged, at least partly, above the tongue 5 in expanded state of the clamping device 1.

### Exemplary embodiments of a connector forming the first clamp end

Referring to Fig. 1, 3A, 5, 7, 9B, 10-12, due to the hollow structure of the second connector section CS2, for instance with a locking transverse passage O1 that has a section elongated rearwardly from the front portion of the bulge B1, the insert 41, 41a of the first connector section CS1 may ensure a robust connection to obtain a connector CC that is vertically thick at least at the front side thereof.

The transverse passage O1 and a cavity CH in the connector section CS1, CS2 make the connector CC hollow with two opposite open accesses. Typically, the transverse passage O1 and, optionally the cavity CH or similar access region provided in the connector section CS1, are delimited between lower and upper elongated sides (which may be parallel sides) of the connector CC.

The insert 41 extends inside the upper bulge B1, thus being at least partly arranged above the tongue 5. Referring to Fig. 1, the insert 41, 41a may have an insertion size L41 that is lower than insert length L as measured along an extension direction D1 of the first clamping jaw 11, which is an extension direction going away from the rear opening 31. Optionally, length of the fixing region RF1 may have such length L, with the top of the fixing region RF1 possibly having such length L.

When the second clamp end E2 is enlarged, for instance having a length d2 as viewed from a top view of the device (length d2 can be measured along longitudinal axis A), the length L of insert 41, 41a may be greater, for instance at least twice greater, than this length d2.

The insertion member 42 may be a protrusion having a profile/outer circumference with a thinned side at the opposite from a top of the second clamp end E2. It is understood that the insert 41 may be the sole member amongst the interlocking members, which has an annular section/profile (thus having a central hollow). In option of Fig. 5, two hollow inserts 41a, 41b are provided to cooperate in complementary passages O1, O1' provided in the second connector section CS2 and opening at the joining face JF2. Only these inserts 41a, 41b may have an annular section/profile (here possibly a triangular shape with rounded angles).

In the first body part 101, the first connection section CS1 and other parts participating to the fastening to the second body part 102 may have laterally protruding members, one of which is forming the insert 41, 41a. Each of these protruding members can optionally include a lug or bead that is retained, in a locked state, by an inner shoulder or similar abutment surface provided in a receiving portion of the second body part 102.

Referring to Fig. 1, insert length L of the insert 41 allow distributing insert lugs L1, arranged as small protruding reliefs on outer circumference or outer surface(s) of the insert 41, at different spaced locations, with varying space relative to the front side of the first clamping jaw 11. In the embodiment of Fig. 2A, such lugs are replaced by an annular bead b1. Similar circumferential beads may be provided on protruding members 42 and 43 used for the fastening at the second clamping jaw 12.

The connector CC may be of greater size than the second clamp end E2, with a greater circumference of the insert 41, 41a provided beneath the pressing surface, as compared to any other interconnecting part/member 42, 43 provided in the second clamping jaw 12. Use of ribs, lugs L1, L2, L3 or beads b1, b2, b3 in a body part is of interest to provide a robust fastening, involving inner abutment surface(s) in the complementary body part to prevent disconnection. Also, having a large connector CC with a bulge B1 formed at a front side of the first clamp end E1 helps in creating a significant spacing between the tongue 5 and the pressing surface that extend transverse to the adjacent front side, so that interference or pinching between a user's finger and the tongue is prevented.

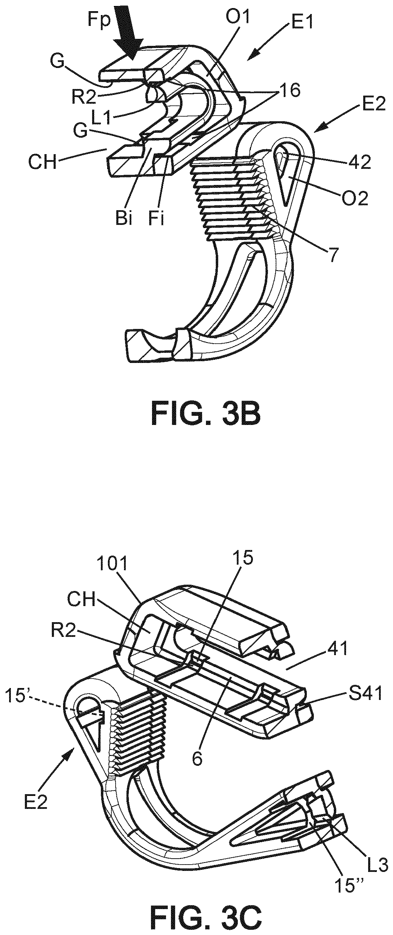

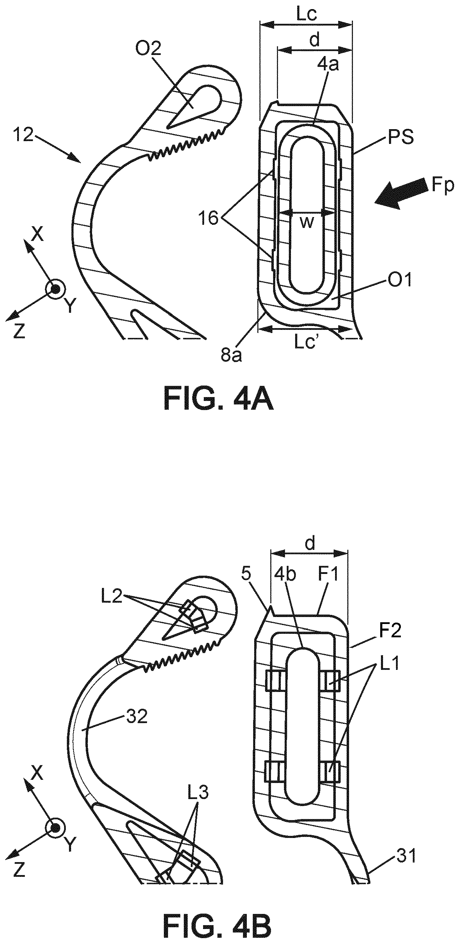

Figs 3C and 4B show an option where each lug L1 may be provided at an axial location between two notches, while radially protruding inwardly. The two notches provide a slanted profile for the lug L1. One of the two notches forms the window 15 so that the slanted lug L1 can be pushed and can be disconnected when pushed according to a direction opposite to the insertion direction. The complementary relief R2 may also be slanted, providing a slope for facilitating disengagement of the lug L1. Similar configuration may be used for engagement of other lugs L2, L3.

In variant shown in Fig. 5, it can be seen the first connector section CS1 may have two laterally protruding members adapted to be engaged inside the hollow region of the second connector section CS2, optionally with a partitioning wall 40 provided in such section CS2 to separate an upper passage O1 from a lower passage O1'.

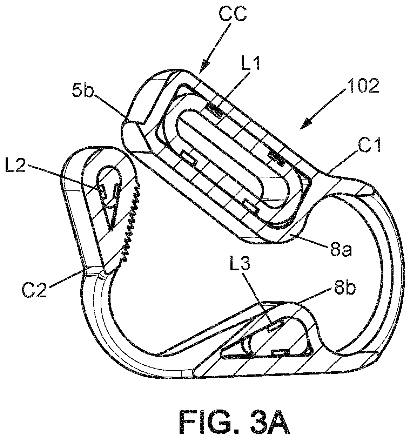

Now referring to Figs 3A-3C, each passage O1, O2 O3 provided for a male or insertion member is delimited by a female member that includes at least two inner reliefs R2 matching with position of corresponding lugs L1, L2, L3 for preventing extraction of the insert 41 and similar insertion members 42, 43 provided at the other fastening regions. In the second connector section CS2, grooves 16, as apparent in Fig. 3B, may be provided for facilitating reception of the lugs L1, L2, L3. But such grooves 16 are delimited by a rim located at or arranged adjacent to a mouth of the corresponding passage O1, O2 or O3. The grooves G, aligned for instance with the grooves 16, are provided in the first connector section CS1 and may be of help to make the relief R2 and the lug L1 more compressible. Indeed, at location of the groove G, the relief R2 is has limited contact with the lug L1. More generally, each lug L1, L2, L3 may correspond to a thinned part in a periphery of the insert or insertion member 41, 42, 43, typically with a thinning obtained using adjacent recesses. For instance, the recesses are provided in an annular base Bi from which the connecting male part of the insert/insertion member protrudes.

A clipping action thus may be obtained without use of fragile protruding parts elongated along the insertion direction.

As a result of using recesses or windows adjacent to the fastening reliefs, when fastening the first body part 101 to the second body part 102, the lugs L1, L2, L3 are elastically deformed when starting insertion to pass over such rim or relief R2 and act as clips, retained in the grooves 16 beyond the corresponding relief R2.

In some options, it may be a light/reversible clipping, for instance with the lugs L1, L2, L3 having beveled or sloped surfaces on both sides, so that the fastening is reversible, possibly with help of a tool.

Such lugs L1, L2, L3 can be provided in a groove area G, 16 or similar recessed part leaving an access to the lugs L1, L2, L3 via dedicated passages or the hollow structure of the first connector section CS1. Such access and design of the lugs allow disconnection of the body parts 101, 102, making use of a tool easier. Such access may be created by having, in the first connector section CS1 (with the insert 41, 41a) and in other connecting parts having the protruding members 42, 43, a hollow construction. A cavity opening at the opposite from the junction plane P can be obtained. For actuating disconnection, when the insertion members 41, 42, 43 are formed adjacent to such a cavity CH, a tool thin end may be interposed between a lug L1, L2, L3 and the corresponding relief R2 to pull and extract the insertion member 41, 42, 43.

The first connector section CS1 may have a variation in section of the inner circumference when having an annular shape. For instance, the insert 41 of annular shape may be sized to be spaced relative to an annular inner face Fi distributed in the first connector section CS1 and the second connector section CS2.

Referring to Figs 3B and 3C, the insert 41 protrudes from an annular base Bi that radially joins/connects the insert 41 to the outer annular wall part of the first connector section CS1. More generally when having a generally annular construction of the connector CC, a junction base Bi may form an inner shoulder 6 of the first connector section CS1, with the insert 41 protruding from the junction base Bi. The junction base Bi may be delimited between the inner shoulder 6 and a shoulder surface S41 from which the insert 41 protrudes.

In some options, the base Bi is slotted or provided with notches/apertures 15, N1, and at least one of the lugs L1 is selectively provided as relief that is spaced from the base Bi and aligned (along insertion direction of the insert 41) with a corresponding slot N1 or aperture 15. Each lug L1 may possibly be provided in an area/angular sector that is complementary from areas/angular sectors where an inner shoulder 6 forming the base Bi extends. Such inner shoulder reduces size of the hollow of the insert 41, as compared to extension of the cavity CH.

The number of lugs L1 may vary, for instance with at least two outer lugs distributed peripherally/around the annular body of the insert 41.

Other connecting parts of the clamping device 1 may have similar lugs L2, L3 provided in insertion members 42, 43 that have a cross section lower than cross section of the insert 41. The insertion members 42, 43 may be not hollow, and possibly provided adjacent to a cavity (similar to cavity CH). The insertion members 42, 43 may be provided in any one of the body part 101, 10, provided that a corresponding/complementary receiving female part is formed in the other body part for anchoring of the insertion member 42 or 43.

Apertures 15' and 15', or notches N2, N3, which are similar to apertures/notches 15, N1 can be provided at a junction base from where an insertion member 42, 43 respectively protrudes. These members 42, 43 can protrude in same direction as the insert 41, as reflected in non-limiting depicted embodiments.

The retaining effect in the receiving female part, for the insert 41 and for the other insertion members 42, 43, can be obtained using a shoulder RB, a relief R2 or any suitable abutment surface. While Figs 2A-2B and 11A use one or more insertion members 41, 42, 43 that have wide reliefs, for cooperating with one or more shoulders RB, for instance a peripheral shoulder (as apparent in Fig. 2B) or a pair of parallel shoulders (as apparent in Fig. 11A), any abutment surface may be formed to allow the retaining effect, whatever the way the body parts 101, 102 are constructed and whatever the location of the junction plane P.

### Options for locking the device in assembled state

The second body part 102 may joined to the first body part 101 along the junction plane P, with a junction J, J' where joining faces JF1, JF2 are contacting one each other. The insert 41 and the insertion members 42, 43 may be included in a same body part 101, to protrude from a same joining face JF1. The joining faces JF1, JF2 may be selectively interrupted at location of the rear and front openings.

A guiding of the insert 41 and/or the other insertion member(s) 42, 43 may be provided, for instance using bevelled surfaces SB at an inlet side of the passages O1, O1', O2, O3. Such bevelled surface may be an annular surface, as illustrated in Fig. 2B for instance. Each insert/insertion member 41, 42, 43 may also have a tapered free end. With such arrangement, a guiding and centring effect is obtained. When the clamping device 1 has a hinge connection linking the two body parts, each protrusion/insert may slide against the bevelled surface before a full insertion. In any case, the insert 41 may be fully covered, annularly, by the second connector section CS2. Each lug L1 or bead 1 is engaged onto a corresponding shoulder or abutment surface RB.

Now referring to Figures 4A-4B, it can be seen that two or more lugs L1 may provide an anchoring effect, so that the insert 41 can be inserted via the passage O1 and quickly retained in the hollow region of the second connector section CS2 with distributed clipping contacts, possibly distributed with:
- at least a first retention obtained close to the clamping part 8a, and
- at least a second retention obtained vertically away (at a greatest vertical distance) from the clamping part 8a,
each retention being enabled against an annular inner face of the connector second section CS2. For ease of insertion, an end 4a of the insert 41, distal form the base Bi, may be provided without any relief (as apparent in Fig. 4A), while the lugs L1 are formed close or adjacent to the base Bi (see Figs 3A-3B and 4B).

Embodiments with three or more lugs L1 can be provided for instance for a clamping device 1 able to be clamped on a hose of a diameter superior to 6 or 7 mm. When having a lower section for the openings 31, 32, for instance to cooperate with a hose diameter inferior or equal to 6 mm, the number of lugs L1 may optionally be only two, for instance as in the embodiment of Fig. 9A.

In embodiments with recesses, windows or similar notches/apertures 15, 15', 15", the locking can be reversible. A light clipping can be obtained by combining bevelled engagement surface in the lugs L1, L2, L3 with bevelled surface (in protruding relief R2) forming the inner shoulder 45 in the second connector section CS2. A retaining effect is obtained by combining at least two lug engagements at opposite sides of the insert 41 and/or insertion member 42, 43.

In contrast, when having clips 51 without slanted surface at abutment interface (as in Fig. 11A), which are fully covered by the second connector section CS2, with typically other insertion members clipped, the locking is stronger and may be considered irreversible. In some options, the insertion members 42, 43 may be deprived from reliefs or lugs, thus being only guided when inserted along same direction than the insert 41. When the insert 41 is composed of two parallel clips 51, the locking is robust due to length of the clips along direction D1 of the connector CC (which may substantially parallel to direction D5 of extension the tongue 5). The insert 41 may include two spaced clips 51 that have same protruding extension L41. Of course, any kind of snapping connection may be used, for instance for forming the connector CC. A permanent or removable snapping may be used.

Referring to Fig. 11B, tabs T1, T2 may be provided as extensions in the clips 51, thus allowing a grasping to release the connection. In locked/assembled state, these tabs are available at the opposite from the first connector section CS1, protruding beyond the passage(s) O1, OA' of the second connector section CS2. Access to the tabs T1, T2, allow pinching the clips 51, so that the clamping device 1 is reopenable to open the junction (median junction J or offset junction J'). In other variants, a window or opening (for instance a top window along junction J or J') may allow such disconnection, possibly giving access to an engagement region for the insert 41.

To ensure correct position of the tube or hose 2 to be pinched, at least one amongst the sides or branches 9a, 9b is provided offset from the joining faces JF1 or JF2 provided in the corresponding body part 101, 102. Use of an upper bulge B1 formed by two partial bulges HB has no impact for suitably placing a hose segment of hose 2, before the assembling, between the base segment BS1 and the first connector section CS1 (in expanded state of the first body part 101).

### Exemplary geometry to form the upper bulge

The bulge in first clamp end E1 belongs to a hollow arrangement/connector CC whose upper face defines the pressing surface PS. The hollow arrangement may have a profile, as considered in a side view, with:
- two long sides (for instance with a lower side extending to the clamping part 8a formed at a rear end thereof, while the upper side is extending rearward from the bulge B1 to a camber region C1); and
- two short sides that are comparatively shorter.

The elongated hollow arrangement/connector CC may thus have a thickness Lc or radial extension, as measured between the pressing surface PS and the lower side face facing the second clamping jaw 12, which is greater than protruding extension e5 of the tongue 5.

Whatever the construction of the body of the clamping device 1, the first clamp end E1 may have a significant extension size d (as illustrated in Figs 4A-4B) above the protruding tooth or tongue 5, which is a height extension. This size d may be measured parallel to direction D2 of extension of the toothed section TS. In some embodiment, the relationship d/Lc ≥ 0.5 may be verified (possibly with d/Lc < 0.9), as apparent in Figs 4A-4B and 10 for example. The first clamp end E1 may optionally be provided with a C-shaped or U-shaped section due to the hollow that separates an upper branch or side allowing forming the pressing surface PS from a lower branch or side that extends toward the first clamping part 8a.

The connector CC has a front end (forming the front face F1), which substantially coincides with the first clamp end E1, forming a first short side of the connector CC, while the first clamping part 8a is arranged at a second short side of the connector C, opposite to the first short side.

As apparent for instance in Fig. 4A, extension Lc may be superior or equal to extension Lc', where Lc is extension/length of the first short side, while Lc' is extension/length of the second short side.

The insert 41, 41a is arranged as inner part of the connector CC, with this insert 41, 41 located adjacent to the upper outer face F2 delimiting a top of the bulge B1. The first clamp end E1, which is configured as front portion of the connector CC, defines a front face (face F1) of the bulge angled relative to the face F2 (and thus typically angled relative to pressing surface PS).

The two connector sections CS1, CS2 may form a large hollow due to the upper bulge B. While a generally rectangular section of the connector CC may be provided, as shown in Fig. 1, 5, 7. 10 or 11A for instance, other annular shapes may be provided. For example, a geometry with a lower side shorter that an upper side of the connector CC may be used, possibly using a slanted face forming a transition between the connector lower side and a free edge of the tongue 5.

More generally, one or more slanted or rounded faces may be provided at the first and second clamp ends to allow for a lowering trajectory of the connector CC when pushed at the pressing surface PS without interference between the clamp ends E1, E2 (despite enlargement due to the upper bulge B1 at the first clamp end E1 and optional enlargement of the second clamp end E2). At the opposite from the bulge B, the first clamp end E1 can thus include a slanted portion. The front face F1 of the connector CC has a height that may be superior to 5 mm above the tongue 5, being understood the tongue 5 can be formed at a lower end of the front face F1.

Thanks to the upper bulge B1, the insert 41 may have a width or height extension w of at least 4 mm and a length L greater than the width or height extension w, as illustrated in Fig. 1. With any appropriate section/profile, the insert 41, 41a can extend parallel to the pressing surface PS, with an elongated profile. Width w of the insert 41 may be superior or equal to an interspace height e8 (see Fig. 7), measured perpendicular to the longitudinal axis A, corresponding to minimal spacing delimited between the clamping parts 8a, 8b in expanded state of the clamping device 1.

When having the height or width w constant for the insert 41, at least on half of the length, the insert may have an elongated hollow using sufficient spacing (typically same spacing) between an upper side and a lower side of the insert 41. Optionally, the insert 41 has an oblong cross-section or similar annular section, or two spaced elongated straight portions (as shown in Fig. 11A) spaced by a constant spacing. Use of two parallel upper and lower sides allow providing a great deviation (upward deviation) prevents a flattening of the first clamping jaw when reaching the clamp end E1, so a thumb cannot easily reach the tongue area.

The second clamp end E2 can be more compact than the first clamp end E1. Pushing action is exerted at the pressing surface PS that is facing upwardly. The bulge B1 is possibly not adding any additional width in the clamping device 1.

The bulge B1 is not adding longitudinal length to the first clamping jaw 11, so that the second clamp end E2 may be uncovered in closed position such as shown in Fig. 10. The angled surface at the upper bulge B1, possibly with a rounding, may be formed to have face F1 deviating of about 90° relative to general direction D1 of the first clamping jaw 11 (which may also a longitudinal direction of the connector CC). This allow the front face F1 to be parallel to and directly facing the toothed section TS, in closed state, so that the upper bulge B1 does not interfere with downward movement of the tongue 5 arranged to protrude at a lower end of this face F1. The toothed section TS may define a given direction D2 along which the clamp end E1 is movable to have different closing positions, with an increased pinching effect when the clamp end E1 is lowered: this may allow an adaptation to hose section.

Referring to Fig. 6, it is depicted a biopharmaceutical fluid circuit with a tubing set of the that may include a hose 2, which provides a flow path to a collection container 100. A sampling pouch could also be connected fluidically with such hose 2. A clamping device 1 with the upper bulge B1 and having ability to be assembled from two body parts 101, 102 can easily be located as desired in such fluid circuit. The junction J can be obtained by clipping or any suitable connection, using the insert 41, 41a adjacent to the bulge B. The bulge B1 prevents risk of having the tongue cutting fragile parts or interfering with the user's hand, while enhancing the fixation of the body parts 101, 102.

In some options, the clamping device 1 may be configured so that, once closed, it remains irreversibly closed. An "irreversibly" closed or closable flow control clamp can only be released from the closed and locked position by extraordinary and unintended manipulation of the clamp, including breakage of the clamp. A clipping may be involved in such situation to quickly assemble the device 1.

For ease at exerting the pressing along direction Fp and displacing the tongue 5, the connector CC may include material that is robust, for instance a rigid or semi-rigid plastic. In some embodiments, each body part 101, 102 is constructed as a single piece of plastic material, preferably molded.

In some embodiments, the insert 41 may have a protruding extension L41 that is different from extension of the insertion members 42, 43. When having a hinge connection 30 arranged in the second clamping jaw 12, the insert 41 may protrude from the joining face JF1 more than insertion member 42 provided in the second clamp end E2 and than another insertion member 43 arranged adjacent the lower clamping part 8b. Fig. 12 illustrates such arrangement. A difference in protruding size may be also present in other embodiments, when having the opening O1, O1' that opens on two sides in the second body part 102, typically when having the body parts 101, 102 of same or similar width as measured along left-right direction (transverse direction of the clamping device 1). Arrangement with insertion extension as in Fig. 12 or similar extension may provide such difference in protruding size.

More generally, use of an insert 41, 41a, 41b with insert parts distributed at different distances from the hose passageway allow, at fixation region RF1, a strengthening of the fixture between the body parts 101, 102, while the other fixation regions may of shorter size. Thickness corresponding to height extension w of the insert 41 may be larger than a height provided for the insertion member 42. It is understood that, along a clamping direction, forming a substantially vertical direction along Z-axis, size/height of the transverse passage O2 is narrower and smaller in section than the passage O1.

Of course, other variations and modifications can easily be made to obtain the assembling of the two body parts. Mechanical connection may be partly or entirely modified to include a locking mechanism in some embodiments. For instance, an interior push-push button or mechanism may be included inside the hollow of the first connector section, allowing locking and unlocking the fixation, with the pushing actuation requiring a pushing exerted (by a finger or tool for example) inside the first clamping jaw 11. In such specific option, access to the pushing (second pushing) for the unlocking may be more difficult and/or partly concealed by a closure/covering member, to prevent accidental disassembling of the clamping device.

While embodiments are shown with each axial opening 31, 32 delimited by a circumference (at the branches 9a, 9b for the rear opening 31) with or without an offset of the junction plane, without any deformation of such circumference, a clamping device 1, may also be provided with a least one of the body parts 101, 102 having a flexible part suitable to adjust section of an opening, for example in the rear opening 31. Additionally, or alternatively, such adjustment may be provided at the front opening 32. Fig. 9B illustrates a non-limiting embodiment with two branches or arms 61, 62 suitable for accepting a hose 2 of reduced cross section as compared to section of the hose passageway as defined by the rear and front openings 31, 32. The guiding branches/arms 61, 62 may guide hose segment inside a partial hose passageway, here formed by the first body part 101, before the assembling. A bent geometry can used for at least one branch 61, 62, so that a large hose can also be received without significant pinching effect. When using two guiding branches 61, 61, allowing adjustment to different hose sizes, they may be joined to a same opening side, away from the junction J, J'. They may be relatively short and typically without protruding beyond the joining face JF1.

The branches 61, 62 may form arched blades or similar flexible tabs, able to hold a hose of reduced section, for instance of about twice smaller size that an inner diameter or maximal size defined at one of the openings/passages 31, 32. Possibly, the rear opening 31 may be provided with two flexible C-shaped branches that protrude from a same side with a divergent bending. More generally each branch 61, 62 can be bent outwardly. Use of a pair of branches 61, 62 may prevent deviation or radial motion of a tube/hose of size smaller than the passage characteristic diameter, with a centering effect.

The branches 61, 62 can be provided in a body part that includes the gripping edges b1, b2 to stabilize/retain the hose 2 inside this body part, before the assembling operation, between the base segment BS1 or BS2 and the connector section CS1 or CS2. Of course, the section adjustment features can be used in many embodiments, with or without the junction plane J, J' offset (and with or without the gripping edges b1, b2). The kind of connection shown in the device 1 of Fig. 9A-9B for assembling the body parts 101, 102 may be modified, provided that a robust fastening is obtained at junction of the connector sections CS1, CS2 that define the bulge with an upwardly deviated pressing surface PS.

Any reference sign in the following claims should not be construed as limiting the claim. It will be obvious that the use of the verb "to comprise" and its conjugations does not exclude the presence of any other elements besides those defined in any claim. The word "a" or "an" preceding an element does not exclude the presence of a plurality of such elements.

## Claims

1. A clamping device (1) for regulating the flow of liquid passing through a flexible hose (2), comprising a clamp body having a longitudinal axis (A) defining a front-rear direction, wherein the clamp body comprises:
- a rear opening (31) and a front opening (32), which are opposite along the longitudinal axis (A) and are forming a hose passageway for receiving the flexible hose (2);
- a pair of clamping jaws (11, 12) each provided with a clamping part (8a, 8b) protruding in the hose passageway in a closed position of the clamping device (1);
wherein interacting securing elements are distributed at front clamp ends (E1, E2) of the clamping jaws and are mutually engaged to anchor the clamping jaws (11, 12) in the closed position, the clamping jaws comprising a first clamping jaw (11) and a second clamping jaw (12) extending both from a bendable connection (4) to a corresponding front clamp end (E1, E2), the first clamping jaw (11) having a pressing surface (PS) for actuation of the device (1) and being downwardly movable in response to a pushing action at the pressing surface (PS) that is facing upwardly and formed in an upper outer face (F2) of the device (1);
wherein the clamp body is an assembly consisting of a first body part (101) and a second body part (102) connectable by fastening means offset relative to the hose passageway,
wherein the first and second body parts form a left body part and a right body part, allowing delimiting left sides and right sides, respectively, of the rear and front openings (31, 32) once the first body part (101) and the second body part (102) are assembled together, the clamp body including a longitudinal junction (J; J') for junction of the first and second body parts in an assembled state that can be obtained after placing a hose segment of the flexible hose in an interspace between said left sides and right sides,
wherein the clamp end (E1) of the first clamping jaw (11), forming a first clamp end (E1), comprises a tongue (5) and a hollow upper bulge (B1) offset upwardly relative to the tongue (5), the first clamp end (E1) having two complementary parts or halves with one half arranged at a front end of a first connector section (CS1) and the other half arranged at a front end of a second connector section (CS2) that delimits a transverse passage (O1), the first connector section (CS1) including an insert (41; 41a) configured to project inside a hollow region of the second connector section (CS2) via the transverse passage (O1) so that the insert (41) extends inside said upper bulge (B1), the tongue (5) protruding at the opposite from the rear opening (31) and being one of the interacting securing elements,
and wherein the first connector section (CS1) belong to the first body part (101) and the second connector section (CS2) belongs to the second body part (102).

2. The clamping device according to claim 1, wherein the first clamp end (E1) is included in a connector (CC) consisting of the first and second connector sections (CS1, CS2) in assembled state, the connector having a generally annular or rectangular periphery with lower and upper elongated sides, the transverse passage (O1) being delimited between the lower and upper elongated sides.

3. The clamping device according to claim 2, wherein the lower elongated side of the connector extends from the clamping part (8a) of the first clamping jaw (11) to the tongue (5).

4. The clamping device according to claim 2 or 3, wherein the insert (41) comprises two lugs or is of annular shape, so that the insert (41) delimits an inner hollow space that opens on at least one side of the clamping device (1),
and wherein the connector (CC), which provides in assembled state the upper pressing surface (PS) for actuating a closed position of the clamping device (1), is an annular connector, preferably elongated, with an annular shape as viewed from a side of the clamping device (1).

5. The clamping device according to any one of claims 2-4, wherein the first clamping jaw (11) comprises a camber region (C1) adjacent to a rear end of the connector (CC) and arranged between the rear opening (31) and the first connector section, the camber region (C1) providing a deviation allowing the connector (CC) to extend upwardly from a direction of the longitudinal axis (A) by providing a pressing surface (PS) progressively extending upwardly with increased space from the camber region (C1), the pressing surface (PS) being preferably a planar or flat surface.

6. The clamping device according to any one of the preceding claims, wherein the insert (41) has an insertion size (L41) lower than an insert length (L) as measured along an extension direction (D1) of the first clamping jaw (11), which is an extension direction going away from the rear opening (31).

7. The clamping device according to any one of the preceding claims, wherein the clamp body is made of two pieces and comprises three pairs of interlocking elements (41, 42, 43, O1, O2, O3) for securing the two pieces that form the first and second body parts (101, 102), with one of the three pairs including the insert (41) and the transverse passage (O1),
and wherein the bendable connection (4) and delimitations of the two openings (31, 32) are distributed in the two pieces, so that the two pieces can be fastened using the interlocking elements (41, 42, 43, O1, O2, O3) to obtain the clamping device (1) once the flexible hose (2) is already extending between the two pieces.

8. The clamping device according to any one of claims 1-7, wherein the clamping part (8a) of the first clamping jaw (11) is:
- a first clamping part (8a) protruding downwardly; and
- provided between the rear opening (31) and the first clamp end (E1) that is arranged in the first clamping jaw (11) at the opposite from the bendable connection (4);
wherein the first clamp end (E1) comprises a front face (F1), the tongue (5) protruding forward from a lower portion of the front face (F1),
wherein the second clamping jaw (12), which is provided with the front opening (32), defines a lower portion of the clamp body, the front opening (32) extending between a second clamping part (8b) and a second clamp end (E2) that is arranged in the second clamping jaw (12), the second clamping part (8b) forming the clamping part of the second clamping jaw (12) and protruding upwardly toward the first clamping part (8a).

9. The clamping device according to any one of claims 1-7, wherein the clamp end of the second clamping jaw (12) constitutes a second clamp end (E2), the first clamp end (E1) being:
- configured to interact with a rear surface of the second clamp end (E2) by the tongue (5) to anchor the clamping jaws (11, 12) in the closed position, the tongue (5) being preferably secured to a toothed section (TS) forming the rear surface of the second clamped end (E2).

10. The clamping device according to any one of the preceding claims, wherein the longitudinal junction (J') is an asymmetric junction so that a body part amongst the first body part (101) and the second body part (102) has:
- a U-shaped circumference part at the rear opening (31) and at the front opening (32), with at least two non-circular segments (b1, b2) that define two opposite branches in the U-shaped circumference;
- a width that is greater than 55% of a full width of the clamping device (1), as measured along a left-right direction.

11. The clamping device according to any one of claims 1-10, wherein the insert (41) protrudes and extends along an insert direction perpendicular to the longitudinal axis (A) and has an annular cross section as viewed in a longitudinal plane parallel to the junction (J; J'),
and wherein the insert (41) is provided with an annular bead (b1) configured to be retained, in the assembled state, by one or more abutment surfaces (RB; R2) protruding inwardly to locally narrow an inner circumference of the second connector section (CS2) that is annular.

12. The clamping device according to any one of claims 1-10, wherein the insert (41) protrudes and extends along an insert direction perpendicular to the longitudinal axis (A) and has an annular cross section as viewed in a longitudinal plane parallel to the junction (J; J'),
and wherein the insert (41) is provided with several lugs (L1) configured to be retained, in the assembled state, by one or more abutment surfaces (RB; R2) protruding inwardly to locally narrow an inner circumference of the second connector section (CS2) that is annular.

13. The clamping device according to any one of the preceding claims, wherein the insert (41) is protruding at the opposite beyond an inner shoulder (45) provided in the second connector section (CS2).

14. The clamping device according to any one of the preceding claims, wherein the insert (41) has a width (w) and an insertion size (L41) lower than a longitudinal extension (L) of the insert (41), which is a size of the insert measured along the pressing surface (PS), preferably parallel to the pressing surface (PS),
and wherein width (w) of the insert (41) is superior or equal to an interspace height (e8), measured perpendicular to the longitudinal axis (A), corresponding to minimal spacing delimited between the clamping parts (8a, 8b) in expanded state of the clamping device (1).

15. The clamping device according to claim 14, wherein the width (w) is constant and the insert has an oblong cross-section or two spaced elongated straight portions spaced by a constant spacing.

16. Use of the clamping device (1) according to any one of the preceding claims, wherein a hose (2) forming a lumen for passage for a biopharmaceutical fluid is installed to seat against two spaced guiding regions provided in the first body part (101), so that the insert (41) protrudes transversally relative to a hose segment of the hose (2) extending between the two spaced guiding regions,
and wherein the second connector section (CS2), included in the second body part (102), is fastened to the first connector section (CS1) to obtain the assembled state, with the hose (2) extending through the front opening (32) and the rear opening (31) of the clamping device (1), one of the two spaced guiding regions providing a first perimeter portion, forming a perimeter fraction of the front opening (32), the other one of the two spaced guiding regions providing a second perimeter portion forming a perimeter fraction of the rear opening (31), each perimeter fraction representing preferably more than 55% of the opening circumference, respectively for the rear opening (31) and the front opening (32).
